# EUROPEAN PATENT APPLICATION

(11) **EP 2 204 145 A1**
(43) Date of publication of application: **07.07.2010**
(21) Application number: 10250002.2
(22) Date of filing: 04.01.2010
(51) Int. Cl.: A61F 5/00

(54) **Method of using barbed sutures for gastric volume reduction**

(30) Priority: 05.01.2009 US 142511 P; 09.12.2009 US 634308
(71) Applicant: Tyco Healthcare Group LP, North Haven, CT 06473 (US)
(72) Inventor: Viola, Frank, Sandy Hook, CT 06482 (US); Belcheva, Nadya, Hamden, CT 06518 (US)
(74) Representative: Soames, Candida Jane

(57) **Abstract**

A method of gastric reduction is disclosed that includes the steps of: inserting an oral-gastric device into the interior volume of the stomach of a subject; and inserting through the esophagus at least one barbed suture in proximity to the muscularis of the stomach of a subject. The barbed suture (30) has a first end having a pointed configuration (34,340), and a second end. The muscularis comprise at least first and second surfaces that are in at least partially interfacing relationship to one another in the interior volume. The method also includes inserting the first and/or the second end of the barbed suture through the muscularis to form at least first and second pull regions; and pulling the ends to cause the first pull region to move the first surface towards the interior volume and to cause the second pull region to move the second surface towards the interior volume.

## Description

### CROSS-REFERENCE TO RELATED APPLICATIONS

This application claims the benefit of and priority to U.S. Provisional Patent Application No. 61/142,511, filed January 5, 2009, the entire disclosure of which is incorporated by reference herein.

### BACKGROUND

### 1. Technical Field

The present disclosure relates to methods of gastric surgery and more particularly to the field of methods of achieving gastric volume reduction.

### 2. Discussion of Related Art

Methods are known in the art that employ a system of components that are used separately or in combination for reducing stomach volume, bypassing a portion of the stomach and/or small intestines and reducing nutrient absorption in the stomach and/or small intestines. Each of the components can be implanted using minimally invasive techniques, using a transesophageal approach under visualization with a flexible endoscope. Laparoscopic surgical techniques may be used to assist in the implantation of the components and/or for adjunctive therapies in the digestive tract.

An artificial stoma is located in the stomach or lower esophagus that reduces the flow of food into the stomach. The stoma device is introduced transesophageally and implanted under visualization with a flexible endoscope. The stoma may be anchored to the esophageal or stomach wall using sutures, staples, clips or other anchoring mechanisms. Optionally, the stoma may be used in conjunction with gastric suturing, stapling or banding to create a narrow passage for installation of the stoma and/or for reduction of gastric volume. The gastric suturing, stapling or banding may be applied using transesophageal or laparoscopic techniques. A line of gastroplasty sutures or staples may be used to create a small gastroplasty pouch with a narrow passage for installation of the stoma. The gastroplasty sutures or staples may be applied using transesophageal or laparoscopic techniques.

Methods are also known in the art for joining and holding portions of a stomach to each other in the performance of a Nissen fundoplication procedure. The Nissen fundoplication procedure requires grasping the fundus of the stomach at a proximal location and pulling the fundus around the esophagus, wrapping the fundus around the esophagus one time and attaching the proximal stomach to an apposing portion of the stomach.

As can be appreciated from the foregoing, horizontal gastroplasty involves stapling the stomach into a small partition while only leaving a small opening for food to pass from the upper stomach pouch to the lower one. This form of gastroplasty has resulted in very poor long-term weight loss. The vertical banded gastroplasty (VBG) features a pouch based on the lesser curvature of the stomach and a polypropylene mesh band ring around the outlet of the pouch. Since no anastomosis is created, the VBG is associated with a low mortality rate and a low risk of infectious complications. However, the VBG is being performed much less frequently, because long-term studies have shown a prominent rate of weight regain or exacerbation of severe heartburn.

### SUMMARY

To advance the state of the art of gastric volume reduction or gastroplasty, the present disclosure relates to a knifeless surgical procedure for volume reduction of the stomach by applying barbed knotless sutures in a "thread lift" type technique.

More particularly, in one embodiment, the present disclosure relates to a method of gastric reduction that includes the steps of: inserting an oral-gastric device into the interior volume of the stomach of a subject; and inserting through the esophagus at least one barbed suture wherein the inserting of the oral-gastric device enables the at least one barbed suture to be positioned in proximity to the muscularis of the stomach of a subject. The at least one barbed suture has a first end having a pointed configuration, and a second end. The muscularis comprise at least first and second surfaces that are in at least partially interfacing relationship to one another with respect to the interior volume of the stomach. The method also includes inserting at least one of the first end and the second end of the at least one barbed suture through the muscularis to form at least first and second pull regions; and pulling the at least one of the first end and the second end of the at least one barbed suture to cause the first pull region to move the first surface towards the interior volume and to cause the second pull region to move the second surface towards the interior volume, reducing thereby the interior volume of the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed until the first and second surfaces move to an at least proximate position one to another, reducing thereby the interior volume of the stomach.

In one embodiment, the method may be performed wherein the step of inserting the at least one of the first end and the second end of the at least one barbed suture through the muscularis to form at least first and second pull regions is performed by inserting the first end of the at least one barbed suture through the muscularis and the serosa to form at least first and second pull regions.

In one embodiment, the method may be performed wherein the step of inserting the at least one of the first end and the second end of the at least one barbed suture through the muscularis to form at least first and second pull regions is performed by inserting the first end of the at least one barbed suture through the first surface, wherein the first end of the at least one barbed suture enters the first surface of the muscularis and exits the first surface of the muscularis and penetrates into the interior volume of the stomach; and inserting the first end of the at least one barbed suture that has penetrated the interior volume of the stomach into the second surface of the muscularis, wherein the first end of the at least one barbed suture enters the second surface of the muscularis and exits the second surface of the muscularis and penetrates into the interior volume of the stomach.

In one embodiment, the method may be performed wherein the step of inserting the at least one of the first end and the second end of the at least one barbed suture through the muscularis to form at least first and second pull regions is performed by inserting a bi-directional barbed suture in the muscularis layers in a series sequential uncrossed configuration before reducing the interior volume of the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed by pulling the barbed suture that has been inserted in the muscularis layers in a series sequential uncrossed configuration to reduce the interior volume of the stomach.

In one embodiment, the method may be performed wherein the step of inserting the at least one of the first end and the second end of the at least one barbed suture through the muscularis to form at least first and second pull regions is performed by inserting a bi-directional barbed suture in the muscularis layers in a cross-pull series sequential configuration before reducing the interior volume of the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed by pulling the barbed suture that has been inserted in the muscularis in a cross-pull series sequential configuration to reduce the interior volume of the stomach.

In one embodiment, the method may be performed wherein the step of inserting the at least one of the first end and the second end of the at least one barbed suture through the muscularis to form at least first and second pull regions is performed by inserting a bi-directional barbed suture in the muscularis in a partially uncrossed and partially cross-pull series sequential configuration before reducing the interior volume of the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed by pulling the at least one barbed suture that has been inserted in the muscularis in a partially uncrossed and partially cross-pull series sequential configuration to reduce the interior volume of the stomach.

In one embodiment, the method may be performed wherein the step of inserting the at least one of the first end and the second end of the at least one barbed suture through the muscularis to form at least first and second pull regions is performed by inserting first and second bi-directional barbed sutures in the muscularis each in a series sequential configuration and in traversing paths before reducing the interior volume of the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed by pulling the first and second bi-directional barbed sutures that have been inserted in the muscularis each in series sequential configuration and in traversing paths to reduce the interior volume of the stomach.

In one embodiment, the method may be performed wherein the step of inserting the at least one of the first end and the second end of the at least one barbed suture through the muscularis to form at least first and second pull regions is performed by inserting a mono-directional barbed suture having an anchor at the trailing end of the suture in a series sequential configuration partially through the muscularis and partially through both the muscularis and the serosa before reducing the interior volume of the stomach wherein the anchor is disposed in the interior volume of the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed by pulling the mono-directional barbed suture having an anchor at the trailing end of the suture that has been inserted in a series sequential configuration partially through the muscularis and partially through both the muscularis and the serosa reducing thereby the interior volume of the stomach wherein the anchor is disposed in the interior volume of the stomach.

In one embodiment, the method may be performed wherein the step of inserting the at least one of the first end and the second end of the at least one barbed suture through the muscularis to form at least first and second pull regions is performed by inserting a mono-directional barbed suture having an anchor at the trailing end of the suture in a series sequential configuration partially through the muscularis and partially through both the muscularis and the serosa before reducing the interior volume of the stomach wherein the anchor is disposed externally to the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed by pulling the mono-directional barbed suture having an anchor at the trailing end of the suture that has been inserted in a series sequential configuration partially through the muscularis and partially through both the muscularis and the serosa reducing thereby the interior volume of the stomach wherein the anchor is disposed externally to the stomach.

In one embodiment, the method may be performed wherein the step of inserting the at least one of the first end and the second end of the at least one barbed suture through the muscularis to form at least first and second pull regions is performed by inserting a substantially mono-directional barbed suture with a bi-directional trailing end in a series sequential configuration through both the muscularis and the serosa before reducing the interior volume of the stomach.

In one embodiment, the method may be performed wherein the step of pulling the at least one of the first end and the second end of the at least one barbed suture to cause the first pull region to move the first surface towards the interior volume and to cause the second pull region to move the second surface towards the interior volume is performed by pulling the substantially mono-directional barbed suture with a bi-directional trailing end in a series sequential configuration through both the muscularis and the serosa reducing thereby the interior volume of the stomach.

In one embodiment, the method may be performed wherein the pointed configuration of the first end of the at least one barbed suture is availed by a surgical needle being removably attached to the at least one barbed suture at the first end thereof.

In one embodiment, the method may be performed wherein the pointed configuration of the first end of the at least one barbed suture is availed by a surgical needle being removably attached to the at least one barbed suture via an aperture formed in the surgical needle at a central portion thereof.

### BRIEF DESCRIPTION OF THE DRAWINGS

Various embodiments of the subject method are described herein with reference to the drawings wherein:

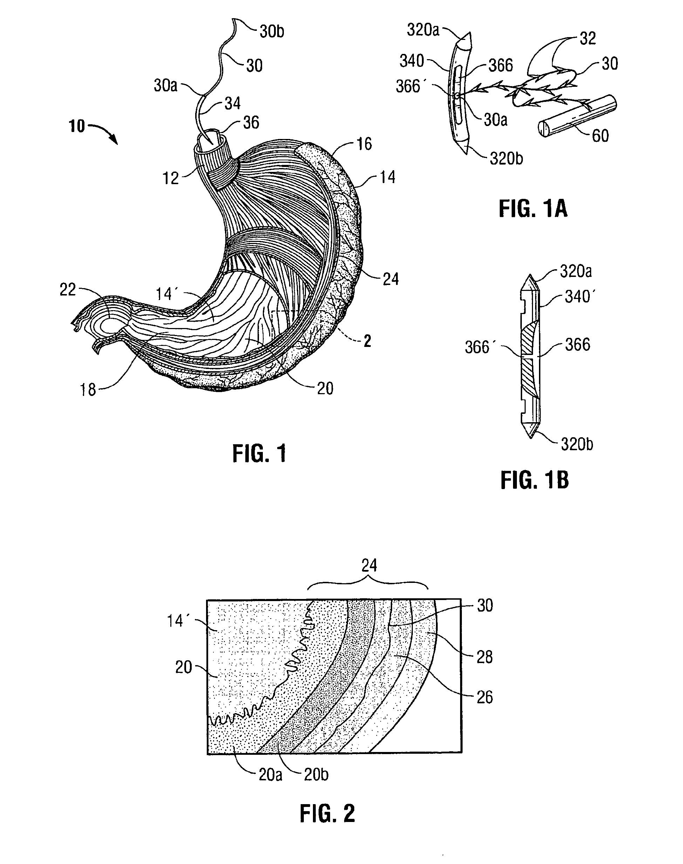

FIG. 1 is a partial view of the digestive tract with a partial view of the interior volume of the stomach;

FIG. 1A is a perspective view of a double-ended curved needle that can be applied to perform the methods of the present disclosure;

FIG. 1B is a view of a double-ended straight needle that can be applied to perform the methods of the present disclosure;

FIG. 2 is a cross-sectional detail view of Detail 2 of the layers of the wall of the stomach of FIG. 1 illustrating a barbed suture in the muscularis layers according to a method of the present disclosure;

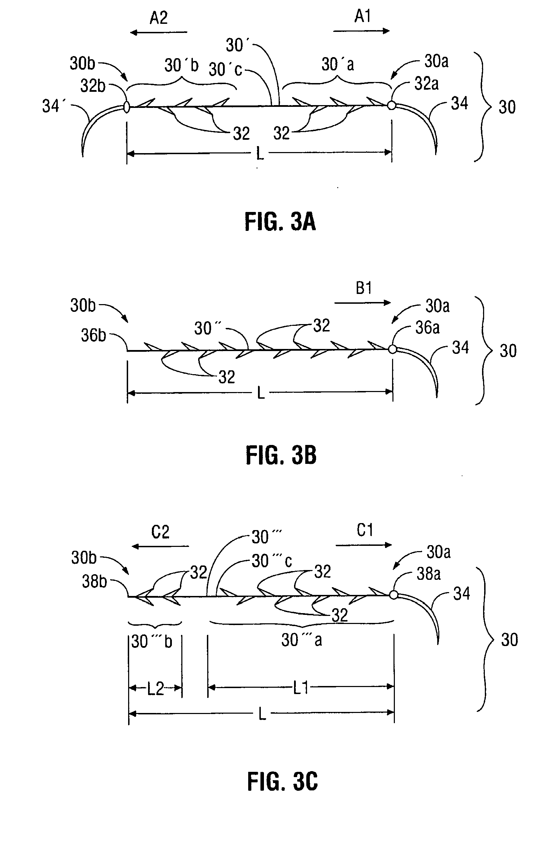

FIGS. 3A-3C are views of various types of barbed sutures with one or two needles attached that may be employed to implement the methods of the present disclosure;

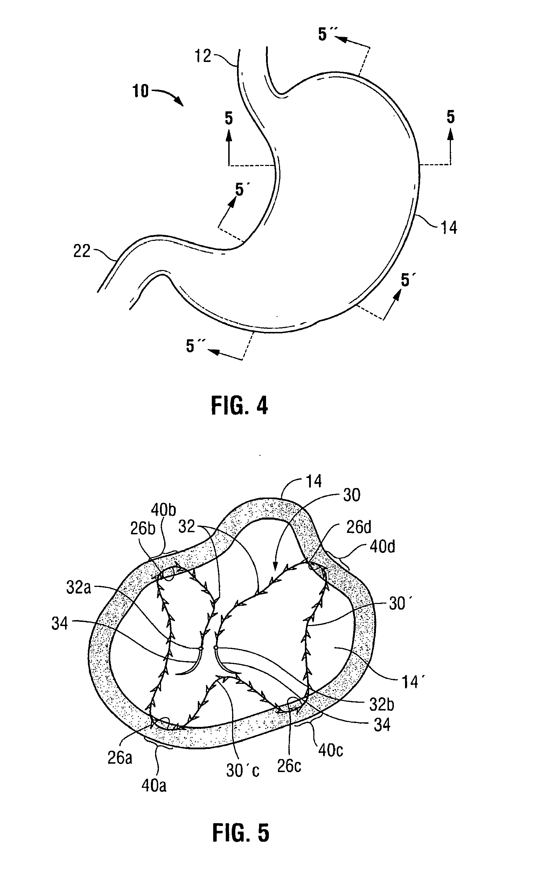

FIG. 4 is a partial view of the digestive tract showing the locations in the stomach for vertical gastroplasties and a horizontal gastroplasty according to various embodiments of the method of the present disclosure;

FIG. 5 is a cross-section view of the stomach at the locations for vertical gastroplasties and a horizontal gastroplasty according to one embodiment of a method of the present disclosure after a bi-directional barbed suture has been inserted in a series sequential uncrossed configuration in the muscularis layers before reducing the interior volume of the stomach;

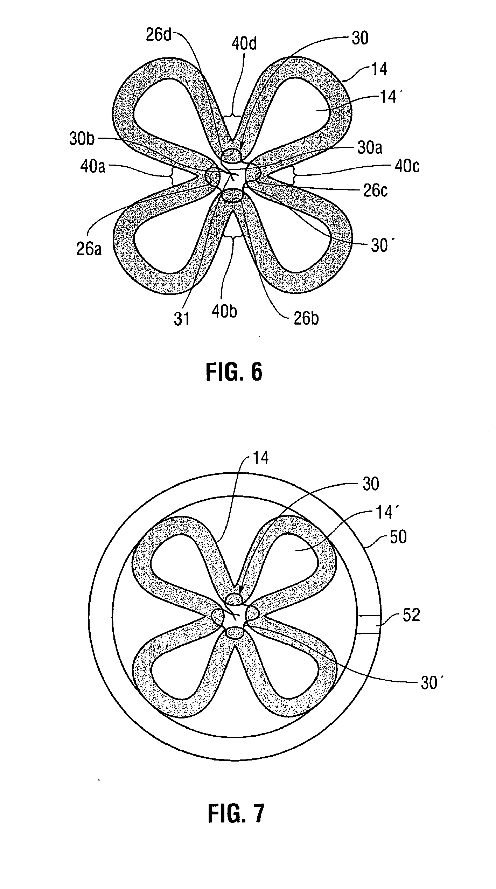

FIG. 6 is a cross-section view of the stomach at the locations for vertical gastroplasties and a horizontal gastroplasty according to one embodiment of a method of the present disclosure after pulling the barbed suture of FIG. 5 that has been inserted in the muscularis layers in a series sequential uncrossed configuration to reduce the interior volume of the stomach;

FIG. 7 is a cross-section view of the vertical gastroplasties and a horizontal gastroplasty according to one embodiment of the method of the present disclosure as illustrated in FIG. 6 and wherein the gastroplasty has been enclosed via a band;

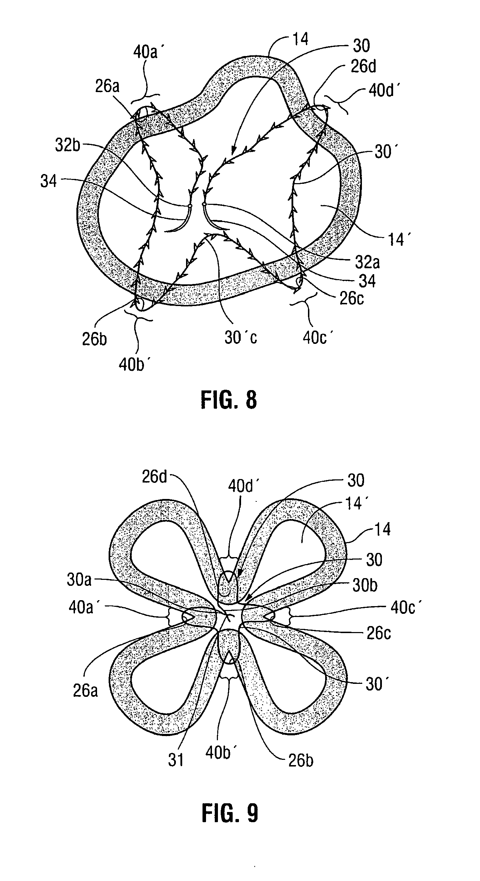

FIG. 8 is a cross-section view of the stomach at the locations for vertical gastroplasties and a horizontal gastroplasty according to one embodiment of the method of the present disclosure after a bi-directional barbed suture has been inserted in the muscularis layers and the serosa in a series sequential uncrossed configuration before reducing the interior volume of the stomach;

FIG. 9 is a cross-section view of the stomach at the locations for vertical gastroplasties and a horizontal gastroplasty according to one embodiment of the method of the present disclosure after pulling the barbed suture of FIG. 8 that has been inserted in the muscularis layers and the serosa in a series sequential uncrossed configuration to reduce the interior volume of the stomach;

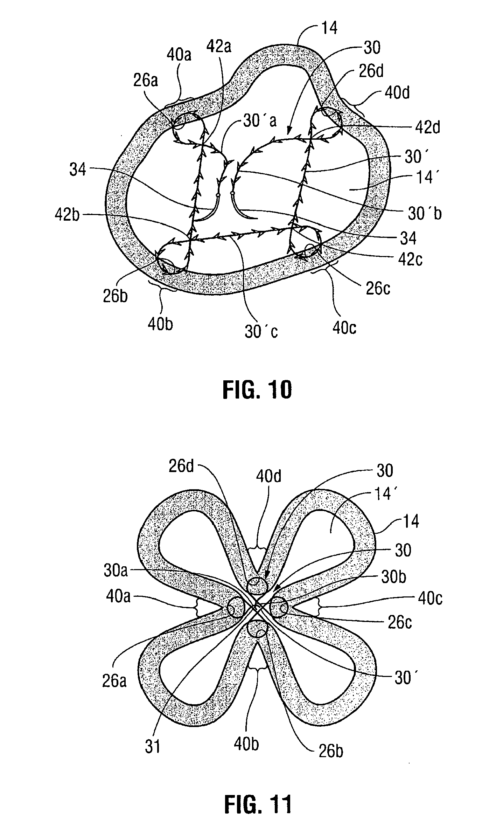

FIG. 10 is a cross-section view of the stomach at the locations for vertical gastroplasties and a horizontal gastroplasty according to one embodiment of the method of the present disclosure after a bi-directional barbed suture has been inserted in the muscularis in a cross-pull series sequential configuration before reducing the interior volume of the stomach;

FIG. 11 is a cross-section view of the stomach at the locations for vertical gastroplasties and a horizontal gastroplasty according to one embodiment of the method of the present disclosure after pulling the barbed suture of FIG. 10 that has been inserted in the muscularis in a cross-pull series sequential configuration to reduce the interior volume of the stomach;

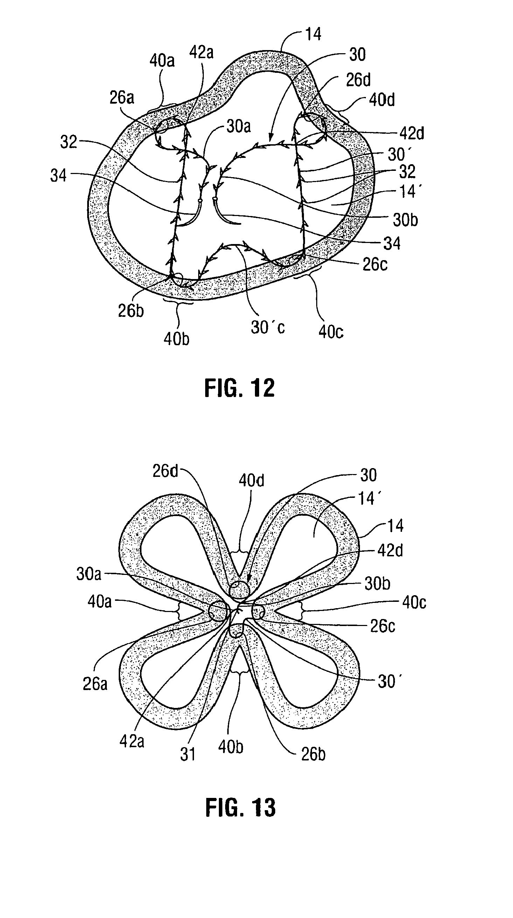

FIG. 12 is a cross-section view of the stomach at the locations for vertical gastroplasties and a horizontal gastroplasty according to one embodiment of the method of the present disclosure after a bi-directional barbed suture has been inserted in the muscularis in a partially uncrossed and partially cross-pull series sequential configuration before reducing the interior volume of the stomach;

FIG. 13 is a cross-section view of the stomach at the locations for vertical gastroplasties and a horizontal gastroplasty according to one embodiment of the method of the present disclosure after pulling the barbed suture of FIG. 10 that has been inserted in the muscularis in a partially uncrossed and partially cross-pull series sequential configuration to reduce the interior volume of the stomach;

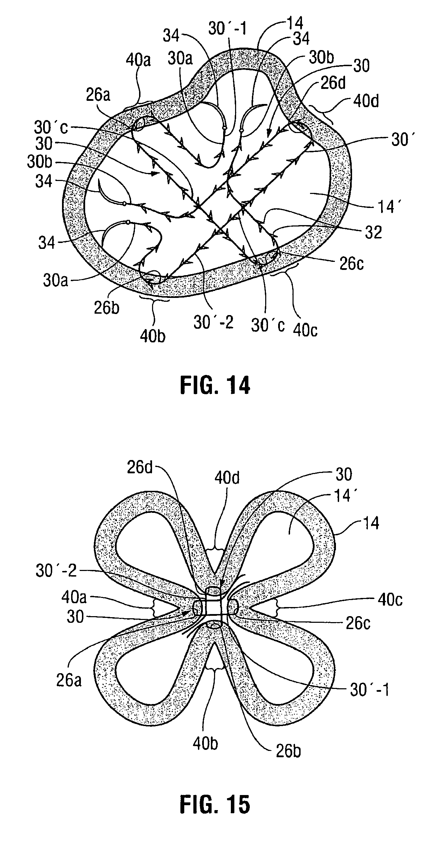

FIG. 14 is a cross-section view of the stomach at the locations for vertical gastroplasties and a horizontal gastroplasty according to one embodiment of the method of the present disclosure after first and second bi-directional barbed sutures have been inserted in the muscularis each in a series sequential configuration and in traversing paths before reducing the interior volume of the stomach;

FIG. 15 is a cross-section view of the stomach at the locations for vertical gastroplasties and a horizontal gastroplasty according to one embodiment of the method of the present disclosure after pulling the first and second bi-directional barbed sutures of FIG. 14 that have been inserted in the muscularis each in series sequential configuration and in traversing paths to reduce the interior volume of the stomach;

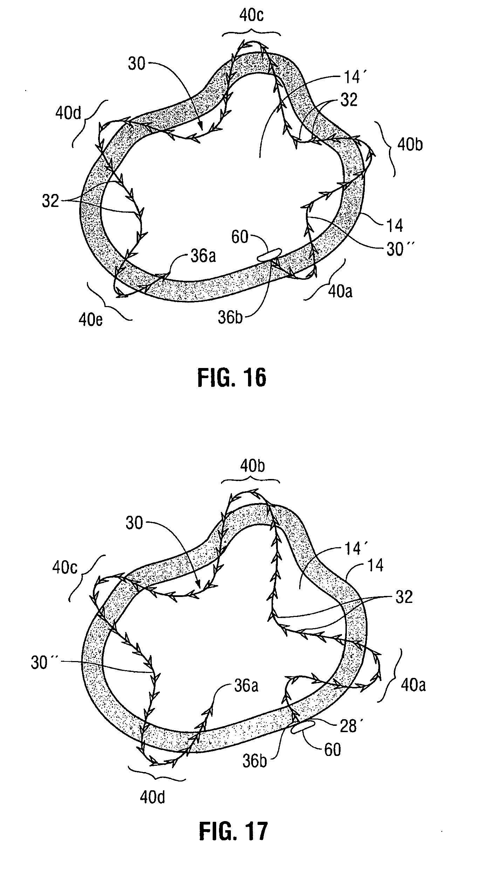

FIG. 16 is a cross-section view of the stomach at the locations for vertical gastroplasties and a horizontal gastroplasty according to one embodiment of the method of the present disclosure after a mono-directional barbed suture having an anchor at the trailing end of the suture has been inserted in a series sequential configuration partially through the muscularis and partially through both the muscularis and the serosa before reducing the interior volume of the stomach wherein the anchor is disposed in the interior volume of the stomach;

FIG. 17 is a cross-section view of the stomach at the locations for vertical gastroplasties and a horizontal gastroplasty according to one embodiment of the method of the present disclosure after a mono-directional barbed suture having an anchor at the trailing end of the suture has been inserted in a series sequential configuration partially through the muscularis and partially through both the muscularis and the serosa before reducing the interior volume of the stomach wherein the anchor is disposed externally to the stomach; and

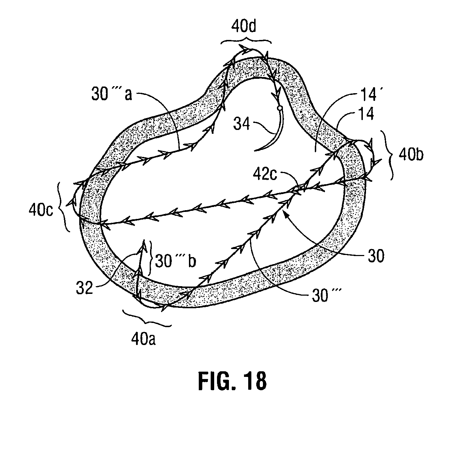

FIG. 18 is a cross-section view of the stomach at the locations for vertical gastroplasties and a horizontal gastroplasty according to one embodiment of the method of the present disclosure after a substantially mono-directional barbed suture with a bi-directional trailing end has been inserted in a series sequential configuration through both the muscularis and the serosa before reducing the interior volume of the stomach.

### DETAILED DESCRIPTION

Embodiments of the present disclosure will now be described in detail with reference to the drawings, in which like reference numerals designate identical or corresponding elements in each of the several views.

In the drawings and in the description that follows, the term "proximal," as is traditional, will refer to the end or portion of a surgical instrument or location of a surgical procedure which is closest to the operator, while the term "distal" will refer to the end or portion of the surgical instrument or location of a surgical procedure which is furthest from the operator.

The present disclosure relates generally, but not exclusively, to methods of knifeless surgery for volume reduction of the stomach by applying barbed knotless sutures in a "thread lift" type technique.

In present disclosure, barbed sutures are applied through the muscularis of the stomach in two perpendicular directions to create a "barbed net". The barbed sutures are inserted through the muscularis either alone or also through the serosa to create "pull regions" in the muscularis and, when applicable or desired, also in the serosa. Thus, creating the "pull regions" makes it possible to "squeeze" the stomach to a smaller interior volume and reduce the volume of possible nutrient intake by the patient or subject compared to the mucosal surface area. The suture barbs are designed to allow only forward motion of the suture into the tissue in the direction of the particular end of the suture. In some embodiments, the suture may comprise monodirectional barbs, i.e., the barbs face a similar direction. In other embodiments, the suture may comprise bidirectional barbs, i.e., the barbs face two distinct directions, enabling both ends of the suture to move forward in more than one direction. Improvements in the design of barbed sutures help to alleviate some of the common knot related problems such as wound healing, restricted blood flow, increased scar formation, and distorted tissue. Another "barbed net" procedure according to the present disclosure may incorporate bands (around fundus and pylorus) for better support of the created gastric structure.

Knifeless surgical procedures for stomach reduction can be beneficial in numerous aspects of bariatric surgery. An advantage of the methods of the present disclosure is that no cutting of the gastric tissue is required, thus potentially reducing the problems of operating on the morbidly obese.

Referring now to FIGS. 1-2, there is illustrated in FIG. 1 a partial view of the digestive tract 10. The esophagus 12, which passes through the diaphragm (not shown), connects to the stomach 14 at the fundus 16. At the pylorus 18, the stomach 14 connects to the duodenum 22. As illustrated at the cut-away section of the stomach wall 24, the stomach 14 has an interior volume 14' that is lined with the rugae 20 or gastric mucous membranes.

As illustrated in FIG. 2, in a direction leading from the interior volume 14', the stomach wall 24 includes the rugae 20. Specifically, the rugae 20 include a first layer 20a, or mucosa, that are disposed lining the interior volume 14', and a second layer 20b, or submucosa, that are adjacent the first layer of mucosa 20a. The rugae 20 are bounded by a third layer 26, known as the muscularis. A fourth layer 28, or serosa, an enclosing serous membrane, forms the outer portion of the stomach wall 24. Stomach wall 24 is illustrated as including the first and second layers 202 and 206, respectively, and third and fourth layers 26 and 28, respectively. In addition, as is discussed in more detail below, the third layer 26 is illustrated with a barbed suture 30 that has been inserted therein according to the methods of the present disclosure.

FIGS. 3A-3B are views of different types of barbed sutures with one or two detachable needles attached that may be employed to implement the methods of the present disclosure. The barbed sutures are generally designated as barbed suture 30. More particularly, there is illustrated in FIG. 3A a bi-directional barbed suture 30' having a length L having a first directional section 30'a on the surface of which barbs 32 are disposed to enable insertion of first end 32a of the suture 30' in tissue of a patient (not shown) in a first direction generally indicated by arrow **A1.** Barbs 32 are also disposed on the surface of a second directional section 30'b to enable insertion of second end 32b of the suture 30' in the tissue of a patient (not shown) in a second direction generally indicated by arrow A2 that is opposite to the direction of arrow **A1.** The bi-directional barbed suture 30' includes a transition section 30'c between the first directional section 30'a and the second directional section 30'b. The transition section 30'c is generally located approximately midway along the length L of the suture 30' and defines a barrier or limit for continued proper directional insertion of the suture ends 32a and 32b in the tissue of a patient. Prior to and during the insertion of the suture ends 32a and 32b in the tissue of a patient, a first curved surgical needle 34 (see also FIG. 1) may be removably attached to the first end 32a and a second curved surgical needle 34' may be removably attached to the second end 32b. It should be understood that although a curved needle is illustrated, various needles may be used and are with the scope of the present disclosure (e.g., straight).

FIG. 3B illustrates a mono-directional barbed suture 30" having a length L extending between first end 36a and second end 36b and on the surface of which barbs 32 are disposed to enable insertion of first end 36a of the suture 30" in tissue of a patient (not shown) only in a direction generally indicated by arrow **B1.** Prior to and during the insertion of the first end 36a in the tissue of a patient, curved surgical needle 34 may be removably attached to the first end 36a.

FIG. 3C illustrates a substantially mono-directional barbed suture 30"' having a length L extending between first end 38a and second end 38b and forming thereby a mono-directional first section 30'''a and a second section 30"'b. Barbs 32 are disposed on the surface defining approximately a length **L1** of the mono-directional first section 30'''a to enable insertion of first end 38a of the suture 30"' in tissue of a patient (not shown) only in a direction generally indicated by arrow **C1.** Barbs 32 are disposed on the surface of second directional section 30"'b in a direction generally indicated by the arrow **C2** that is opposite to the direction of arrow **C1** and only along a length **L2** that is sufficient to cause the second section 30"'b to act as an anchor for the suture 30"' by preventing further movement, in the tissue of a patient, of the suture 30''' in the direction of arrow **C1.** The dimension of length **L2** varies according to the diameter of the suture 30"' and coefficient of friction provided by the material from which the suture 30"' is made with respect to the tissue of the patient but may range from about 1 % of the total length of the suture to about 95 % of the total length of the suture. The substantially mono-directional barbed suture 30"' includes a transition section 30'''c between the first directional section 30"'a and the second directional section 30"'b that defines a barrier or limit for continued proper directional insertion of the suture end 38a in the tissue of a patient. Prior to and during the insertion of the first end 38a in the tissue of a patient, curved surgical needle 34 may be removably attached to the first end 38a.

The barbed sutures 30 (that include barbed sutures 30', 30" and 30"') may be formed of degradable materials, non-degradable materials, and combinations thereof. More particularly, barbed suture 30 may be formed of a degradable material selected from the group consisting of polyesters, polyorthoesters, polymer drugs, polydroxybutyrates, proteins, cat gut, carbonates, homopolymers thereof, copolymers thereof, and combinations thereof. Barbed sutures of the present disclosure may be formed of polymers that are bioabsorbable, and include, but are not limited to polymers selected from the group consisting of aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide)bioabsorbable polymers; and copolymers, block copolymers, homopolymers, blends, and combinations thereof.

More specifically, for the purpose of this disclosure, aliphatic polyesters include, but are not limited to, homopolymers and copolymers of lactide (including lactic acid, D-,L- and meso lactide); glycolide (including glycolic acid); epsilon-caprolactone, p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl-1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof.

Other suitable biodegradable polymers include but are not limited to poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof. Collagen as used herein includes natural collagen such as animal derived collagen, gelatinized collagen, or synthetic collagen such as human or bacterial recombinant collagen.

Suitable polymers which may be used to construct barbed sutures disclosed herein include, for example, synthetic materials, natural materials (e.g., biological) and combinations thereof. Suitable materials include, polyolefins such as polyethylene (including ultra high molecular weight polyethylene) and polypropylene including atactic, isotactic, syndiotactic, and blends thereof; polyethylene glycols; polyethylene oxides; ultra high molecular weight polyethylene; copolymers of polyethylene and polypropylene; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers and copolymers, such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins, such as ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof. Additionally, non-biodegradable polymers and monomers may be combined with each other to create a core of a fiber, for example a fiber possessing a core-sheath configuration.

Barbed suture 30 may be formed using any technique within the purview of those skilled in the art, such as, for example, extrusion, molding and/or solvent casting. In some embodiments, barbed suture 30 may include a yarn made of more than one filament, which may contain multiple filaments of the same or different materials. Where barbed suture 30 is made of multiple filaments, barbed suture 30 may be made using any known technique such as, for example, braiding, weaving or knitting. Barbed suture 30 may also be combined to produce a non-woven suture. Barbed suture 30 may be drawn, oriented, crinkled, twisted, commingled or air entangled to form yarns as part of the suture forming process. In one embodiment, a multifilament suture may be produced by braiding. The braiding may be done by any method within the purview of those skilled in the art. The barbs may be formed utilizing techniques that include, but are not limited to, cutting, molding, etching and the like.

Referring to FIG. 4, the methods of gastroplasty according to the present disclosure may be implemented in at least three different locations in the stomach 14 to enable:
(a) a vertical gastroplasty of the fundus 16 of the stomach 14, as illustrated at section line 5-5;
(b) a vertical gastroplasty of the pylorus 18 of the stomach 14 as illustrated at section line 5'-5'; and (c) a horizontal gastroplasty as illustrated at section line 5"-5".

FIG. 5 illustrates one exemplary embodiment of the method of gastric reduction performed by inserting a bi-directional barbed suture in a series sequential uncrossed configuration according to the present disclosure includes the steps of: inserting an oral-gastric device 36 into the interior volume 14' of the stomach 14 of a subject (see FIG. 1); and inserting through the esophagus at least one bi-directional barbed suture 30, for example by having surgical needles 34 removably attached at first end 32a and second end 32b, wherein inserting the oral-gastric device 36 enables the barbed suture 30 to be positioned in proximity to the muscularis 26 of the stomach 14 of a subject (see FIGS. 1 and 2).

Generically, the barbed sutures 30 each have a first end 30a and a second end 30b having a pointed configuration at at least one end. As illustrated in FIG. 3A-3C, the pointed configuration may be availed by the surgical needle 34 being removably attached to the barbed suture 30 at the first end 32a of suture 30' (see FIG. 3(A)), 36a of suture 30" (see FIG. 3B) or 38a of suture 30"' (see FIG. 3C) and also at the second end 32b of suture 30' (see FIG. 3A).

The surgical needle 34 that avails the pointed configuration may be a curved surgical needle that is suitable for the intended purposes described herein. It is further contemplated that the pointed configuration may also be availed by an endoscopic stitching needle, such as the Endo Stitch™ needle available from Covidien Auto Suture (Norwalk, Connecticut, USA), such as described in U.S. Patent No. 5,478,344 to Stone et al., entitled "SURGICAL SUTURING APPARATUS WITH LOADING MECHANISM", issued Dec. 26,1995, and U.S. Patent No. 5,591,181 to Stone et al., also entitled "SURGICAL SUTURING APPARATUS WITH LOADING MECHANISM", issued Jan. 7, 1997, the entire contents of both of which are hereby incorporated by reference herein. In particular, needle 340 as shown in FIG. 1A is curved, has two pointed ends 320a and 320b and is connected to a portion of barbed suture 30 in the center of needle 340. Channel 366 holds an end, e.g., end 30a, of the barbed suture 30. To retain the barbed suture 30 in the needle 340, the suture 30 may either be glued into channel 366 or the needle itself may be crimped. Alternatively, the barbed suture 30 passes through aperture 366' that is formed in the channel 366 in a central portion of the needle 340 and may be anchored to needle 340 by the barbs 32 of the suture 30 for the duration of the surgical procedure. In addition, a straight needle 340' as shown in FIG. 1B may also be utilized, and the barbed suture 30 (not shown) can be also be connected through the aperture 366'. A single-pointed needle could also be alternatively provided (not shown). As described in more detail below with respect to FIGS. 16 and 17, the opposite end 30b of suture 30 may also have an anchor 60 affixed thereto for securing the suture in tissue.

As illustrated particularly in FIGS. 5 and 6, the muscularis 26 includes at least a first surface 26a and a second surface 26b. The first and second surfaces 26a and 26b of the muscularis 26 are in at least partially interfacing relationship to one another with respect to the interior volume 14' of the stomach 14.

Generically, the method of gastroplasty according to the present disclosure includes the following steps of: inserting at least one end, e.g., the first end and/or the second end of at least one barbed suture through the muscularis 26 to form at least first and second pull regions, before reducing the interior volume 14' of the stomach 14, as described in detail below; and pulling the at least one of the first end and the second end of the at least one barbed suture to cause the first pull region to move the first surface towards the interior volume and to cause the second pull region to move the second surface towards the interior volume, thereby reducing the interior volume 14' of the stomach 14.

More particularly, with respect to FIGS. 5 and 6, the method includes also the following steps: inserting the first end 32a of the barbed suture 30', using, for example, a needle 34 attached to the first directional section 30'a, through the muscularis 26, not further than the transition region 30'c, to form at least first and second pull regions 40a and 40b, respectively; and inserting the second first end 32b of the barbed suture 30', also using, for example, a needle 34 attached to the second directional section 30'b, through the muscularis 26, not further than the transition region 30'c, to form at least third and fourth pull regions 40c and 40d, respectively.

The method may be implemented or performed in more detail by the following steps: inserting the first end 32a of the barbed suture 30' through the first surface 26a wherein the first end 30a enters the first surface 26a and exits the first surface 26a and penetrates into the interior volume 14' of the stomach 14; and inserting the first end 32a of the barbed suture 30' that has penetrated the interior volume 14' of the stomach 14 into the second surface 26b of the muscularis 26. The first end 32a of the barbed suture 30' enters the second surface 26b and exits the second surface 26b and penetrates into the interior volume 14' of the stomach 14.

Generically, the method includes pulling the first end 32a of the barbed suture 30' to cause the first pull region 40a to move the first surface 26a towards the interior volume 14' and to cause the second pull region 40b to move the second surface 26b towards the interior volume 14', thereby reducing the interior volume of the stomach 14. The method may also include pulling the second end 32b of the barbed suture 30' to cause the third pull region 40c to move the third surface 26c towards the interior volume 14' and to cause the fourth pull region 40d to move the fourth surface 26d towards the interior volume 14', thereby reducing the interior volume of the stomach 14.

The method may be implemented or performed in more detail by the following step: pulling the first end 32a until the first and second surfaces 26a and 26b, respectively, move to an at least proximate position one to another, as illustrated in FIG. 6, thereby reducing the interior volume 14' of the stomach 14.

In one embodiment, the method further includes the step of tying the first end 32a of the suture 30' with the second end 32b of the suture 30' to form a knot 31.

In a more detailed implementation of the method, the muscularis 26 includes a third surface 26c. The third surface 26c at least partially interfaces the first and second surfaces 26a and 26b, respectively, of the muscularis 26 with respect to the interior volume 14' of the stomach 14.

The method may be implemented or performed in more detail by the following step of: inserting the first end 32a of the barbed suture 30' through the third surface 26c wherein the first end 32a enters the third surface 26c and exits the third surface 26c and penetrates into the interior volume 14' of the stomach 14.

In a similar manner as described above, the method may be implemented or performed in more detail by the following step of: pulling the first end 32a of the suture 30' until the first, second and third at least partially interfacing surfaces 26a, 26b and 26c, respectively, move to an at least proximate position one to another, thereby reducing the interior volume 14' of the stomach 14.

Again, the method may further include the step of tying the first end 32a of the suture 30' with the second end 32b, thereby forming knot 31. In still another embodiment of the present disclosure, the muscularis 26 include at least first, second, third and fourth surfaces, e.g., first surface 26a, second surface 26b, third surface 26c and a fourth surface 26d at least partially interfacing the first, second and third surfaces 26a, 26b and 26c, respectively, with respect to the interior volume 14' of the stomach 14.

The method further includes the step of: inserting the first end 32a of the barbed suture 30' through the fourth surface 26d of the muscularis 26 wherein the first end 32a of the barbed suture 30' enters the fourth surface 26d and exits the fourth surface 26d and penetrates into the interior volume 14' of the stomach 14.

In a similar manner as described above, again, the method may further include the step of: pulling the first end 32a of the suture 30' until the first, second, third and fourth at least partially interfacing surfaces 26a, 26b, 26c and 26d, respectively, of the muscularis 26 move to an at least proximate position one to another, thereby reducing the interior volume 14' of the stomach 14. Similarly, the method may further include the step of tying the first end 32a with the second end 32b of the suture 30', thereby forming knot 31.

As can be appreciated from the foregoing description with respect to FIGS. 5 and 6, the step of inserting the first end 30a and/or the second end 30b of the one or more barbed sutures 30 through the muscularis 26 to form at least first and second pull regions 40a and 40b is performed by inserting a bi-directional barbed suture 30' in the muscularis 26 in a series sequential uncrossed configuration before reducing the interior volume 14' of the stomach 14.

In addition, the step of pulling the first end 30a and/or the second end 30b of the one or more barbed sutures 30 to cause the first pull region 40a to move the first surface 26a towards the interior volume 14' and to cause the second pull region 40b to move the second surface 26b towards the interior volume 14' is performed by pulling the barbed suture 30' that has been inserted in the muscularis 26 in a series sequential uncrossed configuration to reduce the interior volume 14' of the stomach 14.

Those skilled in the art will recognize that, and understand how, a step of pulling the first end 30a and/or the second end 30b of the one or more barbed sutures 30 to cause the third and fourth pull regions 40c and 40d, respectively, to move the third and fourth surfaces 26c and 26d, respectively, towards the interior volume 14' may also be performed by pulling the barbed suture 30' that has been inserted in the muscularis 26 in a series sequential uncrossed configuration to reduce the interior volume 14' of the stomach 14.

The step of pulling the first end 30a and/or the second end 30b of the one or more barbed sutures 30 to cause the first pull region 40a to move the first surface 26a towards the interior volume 14' and to cause the second pull region 40b to move the second surface 26b towards the interior volume 14', and further optionally including to cause third pull region 40c to move the third surface 26c towards the interior volume 14' and to cause the fourth pull region 40d to move the fourth surface 26d towards the interior volume 14' may be performed until the first and second surfaces 26a and 26b, respectively, move to an at least proximate position one to another, and until the third and fourth surfaces 26c and 26d, respectively, move to an at least proximate position one to another reducing thereby the interior volume 14' of the stomach 14.

Thus, as can be appreciated, referring to FIG. 4, the methods of gastric reduction according to the present disclosure as described above may be performed in a manner wherein the muscularis 26 includes at least first and second at least partially interfacing surfaces 26a and 26b, respectively, and may include the third and fourth at least partially interfacing surfaces 26c and 26d, being disposed within the interior volume 14' of the stomach 14 to enable either: (a) a vertical gastroplasty of the fundus 16 of the stomach 14, as illustrated by section line 5-5 in FIG. 4; or (b) a vertical gastroplasty of the pylorus 18 of the stomach 14, as illustrated by section line 5'-5' in FIG. 4; or (c) a horizontal gastroplasty as illustrated by section line 5"-5" in FIG. 4. Those skilled in the art will recognize that, and understand how, additional surfaces beyond the first through fourth surfaces 26a through 26d, and corresponding first through fourth pull regions 40a through 40d, may be selected by the surgeon as deemed necessary and practical.

In one embodiment of the present disclosure, wherein at least the first and second surfaces 26a and 26b, respectively, and optionally further including the third surface 26c or further the fourth surface 26d, are selected to be disposed within the interior volume 14' of the stomach 14 to enable either: (a) a vertical gastroplasty of the fundus 16, as illustrated in FIG. 4 by section line 5-5; or (b) a vertical gastroplasty of the pylorus 18, as illustrated in FIG. 4 by section line 5'-5', or (c) a horizontal gastroplasty of the stomach 14, as illustrated in FIG. 4 by section line 5"-5", the method may further include the step of either: (a) enclosing the vertical gastroplasty of the fundus 16 via a band 50 as illustrated in FIG. 7 configured and disposed at a position around the serosa 28 with respect to the one or more barbed sutures 30 inserted within the muscularis 26 to impede or prevent re-expansion of the interior volume 14' of the stomach 14; or (b) enclosing the vertical gastroplasty of the pylorus 18 via the band 50 as also illustrated in FIG. 7, respectively, configured and disposed at a position around the serosa 28 with respect to the one or more barbed sutures 30 inserted within the muscularis 26 to impede or prevent re-expansion of the interior volume 14' of the stomach 14. In either scenario, the band 50 may be joined by suitable surgical methods at joint 52. Those skilled in the art will recognize that the band 50 may be positioned using minimally invasive surgical techniques.

FIG. 8 is a cross-section view of the stomach 14 at the locations for vertical gastroplasties and a horizontal gastroplasty described above with respect to FIG. 4 according to one embodiment of the method of the present disclosure after the bi-directional barbed suture 30', described above with respect to FIGS. 5 and 6, has been inserted, in addition to the muscularis 26, also in the serosa 28, in a series sequential uncrossed configuration before reducing the interior volume 14' of the stomach 14.

FIG. 9 is a cross-section view of the stomach at the locations for vertical gastroplasties and a horizontal gastroplasty according to one embodiment of the method of the present disclosure after pulling the bi-directional barbed suture 30' of FIG. 8 that has been inserted, in addition to the muscularis 26, also in the serosa 28 in a series sequential uncrossed configuration to reduce the interior volume 14' of the stomach 14.

Those skilled in the art will recognize that first, second, third and fourth pull regions 40a', 40b', 40c' and 40d', analogous to the first, second, third and fourth pull regions 40a, 40b, 40c and 40d, are now effected by first, second, third and fourth surfaces 26a', 26b', 26c' and 26d', respectively, on the exterior of the serosa, as established at the positions at which the bi-directional barbed suture 30' has been inserted through the serosa 28.

Thus it can be appreciated that the step of inserting the first end 32a and/or the second end 32b of the one or more barbed sutures 30' through the muscularis 26 to form at least first and second pull regions 40a and 40b, respectively, is performed by inserting the first end 32a of the one or more barbed sutures 30' through the muscularis 26 and the serosa 28 to form at least first and second pull regions 40a' and 40b', respectively, that are positioned on the exterior surface of the stomach 14.

Similarly, it can be appreciated that the step of inserting the first end 32a and/or the second end 32b of the one or more barbed sutures 30' through the muscularis 26 to form at least third and fourth pull regions 40c and 40d, respectively, is performed by inserting the second end 32b of the one or more barbed sutures 30' through the muscularis 26 and the serosa 28 to form at least third and fourth pull regions 40c' and 40d', respectively.

Those skilled in the art will recognize that, and understand how, a step of pulling the first end 30a and/or the second end 30b of the one or more barbed sutures 30 to cause the third and fourth pull regions 40c' and 40d', respectively, to move the third and fourth surfaces 26c and 26d, respectively, towards the interior volume 14' may also be performed by pulling the barbed suture 30' that has been inserted in the muscularis 26 in a series sequential uncrossed configuration to reduce the interior volume 14' of the stomach 14.

Again, a step of pulling the first end 30a and/or the second end 30b of the one or more barbed sutures 30 to cause the first pull region 40a' to move the first surface 26a towards the interior volume 14' and to cause the second pull region 40b' to move the second surface 26b towards the interior volume 14', and further optionally including to cause third pull region 40c' to move the third surface 26c towards the interior volume 14' and to cause the fourth pull region 40d' to move the fourth surface 26d towards the interior volume 14' may be performed until the first and second surfaces 26a and 26b, respectively, move to an at least proximate position one to another, and until the third and fourth surfaces 26c and 26d, respectively, move to an at least proximate position one to another, thereby reducing the interior volume 14' of the stomach 14.

The remaining embodiments of the methods of the present disclosure will be described with respect to one or more barbed sutures 30 being inserted only through the muscularis 26 and not also through the serosa 28. However, those skilled in the art will recognize that, and understand how, the remaining embodiments of the methods of the present disclosure may be performed by inserting one or more barbed sutures 30 through both the muscularis 26 and the serosa 28.

Accordingly, FIG. 10 is a cross-section view of the stomach 14 at the locations for vertical gastroplasties and a horizontal gastroplasty according to one embodiment of the method of the present disclosure after a bi-directional barbed suture 30' has been inserted in the muscularis 26 in a cross-pull series sequential configuration before reducing the interior volume 14' of the stomach 14.

FIG. 11 is a cross-section view of the stomach 14 at the locations for vertical gastroplasties and a horizontal gastroplasty according to one embodiment of the method of the present disclosure after pulling the barbed suture 30' of FIG. 10 that has been inserted in the muscularis 26 in a cross-pull series sequential configuration to reduce the interior volume 14' of the stomach 14.

Thus it can be appreciated in view of FIGS. 10 and 11 that a step of inserting first end 26a and second end 26b of one or more barbed sutures 30 through the muscularis 26 to form at least first and second pull regions 40a and 40b, respectively, is performed by inserting bi-directional barbed suture 30' in the muscularis 26 in a cross-pull series sequential configuration before reducing the interior volume 14' of the stomach 14. As defined herein, a cross-pull series sequential configuration refers to a configuration in which overlaps are created in the path of the one or more barbed sutures 30 that are inserted in a series sequential configuration in the muscularis 26 (or through both the muscularis 26 and the serosa 28).

More particularly, the embodiment of the method of present disclosure as illustrated in FIG. 10 is implemented or performed wherein the first directional section 30'a (see FIG. 3A) of the bi-directional barbed suture 30' is inserted in the muscularis 26 to form a first overlap 42a in the vicinity of the first pull region 40a and to form a second overlap 42b in the vicinity of the second pull region 40b. Additionally, the method is implemented or performed wherein the second directional section 30'b (see FIG. 3A) of the bi-directional barbed suture 30' is inserted in the muscularis 26 to form a third overlap 42c in the vicinity of the third pull region 40c and to form a fourth overlap 42d in the vicinity of the fourth pull region 40b. The overlaps 42a, 42b, 42c and 42d enable a cross-pull of the suture 30' in the vicinity of the respective pull regions 40a, 40b, 40c and 40d that may enhance the effectiveness of the method of gastroplasty. As previously mentioned, additional overlaps and pull regions may be formed as necessary.

It may be appreciated that a step of pulling the the first end 30a and/or the second end 30b of the one or more barbed sutures 30 to cause the first pull region 40a to move the first surface 26a towards the interior volume 14' and to cause the second pull region 40b to move the second surface 26b towards the interior volume 14' is performed by pulling the bi-directional barbed suture 30' that has been inserted in the muscularis 26 (or the muscularis 26 and the serosa 26) in a cross-pull series sequential configuration to reduce the interior volume 14' of the stomach 14. Those skilled in the art will recognize that the method may be applied correspondingly to the suture 30' also with respect to the third and fourth pull regions 40c and 40d, respectively.

Again, as illustrated in FIG. 11, a step of pulling the first end 30a and/or the second end 30b of the one or more barbed sutures 30 to cause the first pull region 40a to move the first surface 26a towards the interior volume 14' and to cause the second pull region 40b to move the second surface 26b towards the interior volume 14', and further optionally including to cause third pull region 40c to move the third surface 26c towards the interior volume 14' and to cause the fourth pull region 40d to move the fourth surface 26d towards the interior volume 14' may be performed until the first and second surfaces 26a and 26b, respectively, move to an at least proximate position one to another, and until the third and fourth surfaces 26c and 26d, respectively, move to an at least proximate position one to another thereby reducing the interior volume 14' of the stomach 14.

FIG. 12 is a cross-section view of the stomach 14 at the locations for vertical gastroplasties and a horizontal gastroplasty according to one embodiment of the method of the present disclosure after bi-directional barbed suture 30' has been inserted in the muscularis 26 (or in the muscularis 26 and the serosa 28) in a partially uncrossed and partially cross-pull series sequential configuration before reducing the interior volume 14' of the stomach 14.

FIG. 13 is a cross-section view of the stomach 14 at the locations for vertical gastroplasties and a horizontal gastroplasty according to one embodiment of the method of the present disclosure after pulling the barbed suture 30' of FIG. 10 that has been inserted in the muscularis 26 in a partially uncrossed and partially cross-pull series sequential configuration to reduce the interior volume 14' of the stomach 14.

The method of gastroplasty illustrated in FIGS. 12 and 13 can best be understood by comparing to the method of inserting and pulling bi-directional barbed suture 30' based on the foregoing description with respect to FIGS. 5 and 6, wherein the step of inserting the first end 30a and/or the second end 30b of the one or more barbed sutures 30 through the muscularis 26 to form at least first and second pull regions 40a and 40b and third and fourth pull regions 40c and 40d is performed by inserting a bi-directional barbed suture 30' in the muscularis 26 in a series sequential uncrossed configuration before reducing the interior volume 14' of the stomach 14.

The method of gastroplasty illustrated in FIGS. 12 and 13 can further best be understood by comparing to the embodiment of the method of present disclosure as illustrated in FIG. 10 wherein the first directional section 30'a (see FIG. 3A) of the bi-directional barbed suture 30' is inserted in the muscularis 26 to form a first overlap 42a in the vicinity of the first pull region 40a, and the second directional section 30'b (see FIG. 3A) is inserted in the muscularis 26 to form a fourth overlap 42d in the vicinity of the fourth pull region 40b. The first overlap 42a and the fourth overlap 42d (effectively a second overlap herein) enable a partial cross-pull of the suture 30' in the vicinity of the respective pull regions 40a and 40d, which in combination with the partially uncrossed configuration of the suture 30' in the vicinity of second pull region 40b and third pull region 40c may enhance the effectiveness of the method of gastroplasty.

Thus, the method of gastroplasty illustrated in FIG. 12 is implemented or performed by a step of inserting first directional section 30'a (see FIG. 3A) of the bi-directional barbed suture 30' in the muscularis 26 to form a first overlap 42a in the vicinity of the first pull region 40a and by continuing to insert the bi-directional barbed suture 30' in the muscularis 26 in a series sequential uncrossed configuration to form the second pull region 40b before reducing the interior volume 14' of the stomach 14. The method may also be implemented or performed by a step of inserting first directional section 30'a in the muscularis 26 in a series sequential uncrossed configuration to form the third pull region 40c and by continuing to insert the bi-directional barbed suture 30' in the muscularis 26 to form the fourth overlap 42d in the vicinity of the fourth pull region 40d before reducing the interior volume 14' of the stomach 14.

Those skilled in the art will recognize that, as illustrated in FIG. 12, a step of pulling the first end 30a and/or the second end 30b of the one or more barbed sutures 30 to cause the first pull region 40a, the first overlap 42a being in the vicinity thereof, to move the first surface 26a towards the interior volume 14' and to cause the second pull region 40b to move the second surface 26b towards the interior volume 14', and further optionally including to cause third pull region 40c to move the third surface 26c towards the interior volume 14' and to cause the fourth pull region 40d, the fourth overlap 42d being in the vicinity thereof, to move the fourth surface 26d towards the interior volume 14' may be performed until the first and second surfaces 26a and 26b, respectively, move to an at least proximate position one to another, and until the third and fourth surfaces 26c and 26d, respectively, move to an at least proximate position one to another, thereby reducing the interior volume 14' of the stomach 14.

FIG. 14 is a cross-section view of the stomach 14 at the locations for vertical gastroplasties and a horizontal gastroplasty according to one embodiment of the method of the present disclosure after first and second bi-directional barbed sutures 30'-1 and 30'-2, respectively, have been inserted in the muscularis 26 (or in the muscularis 26 and the serosa 28) each in a series sequential configuration and in traversing paths before reducing the interior volume 14' of the stomach 14.

FIG. 15 is a cross-section view of the stomach 14 at the locations for vertical gastroplasties and a horizontal gastroplasty according to one embodiment of the method of the present disclosure after pulling the first and second bi-directional barbed sutures 30'-1 and 30'-2, respectively, of FIG. 14 that have been inserted in the muscularis 26 (or in the muscularis 26 and the serosa 28) each in series sequential configuration and in traversing paths to reduce the interior volume 14' of the stomach 14.

Thus it may be appreciated that the method of gastroplasty illustrated in FIGS. 14 and 15 includes the step of inserting the first end 30a of the first barbed suture 30'-1, using, for example, needle 34 (see FIG. 3A) attached to the first directional section 30'a, through the muscularis 26, not further than the transition region 30'c, to form at least first pull region 40a and the step of inserting the second end 32b of the first barbed suture 30'-1, again using, for example, needle 34 (see FIG. 3A) attached to the second directional section 30'b, through the muscularis 26, not further than the transition region 30'c, to form at least second pull region 40b.

The method of gastroplasty illustrated in FIGS. 14 and 15 also includes the step of inserting the first end 30a of the second barbed suture 30'-2, using, for example, needle 34 (see FIG. 3A) attached to the first directional section 30'a, through the muscularis 26, not further than the transition region 30'c, to form at least third pull region 40c and the step of inserting the second end 30b of the barbed suture 30'-2, again using, for example, needle 34 (see FIG. 3A) attached to the second directional section 30'b, through the muscularis 26, not further than the transition region 30'c, to form at least fourth pull region 40d.

Generally, but not exclusively, the first and second bi-directional barbed sutures 30'-1 and 30'-2, respectively, may be inserted in traversing paths as shown in FIGS. 14 and 15. In the case of traversing paths, those skilled in the art will recognize that the surgeon implements the method by considering the sequence of insertion beginning from a distal-most location and ending at the most proximal location in the interior volume 14'.

In addition, the step of pulling the first and second bi-directional barbed sutures 30'-1 and 30'-2, respectively, of FIG. 14 that have been inserted in the muscularis 26 (or in the muscularis 26 and the serosa 28) each in series sequential uncrossed configuration may be implemented or performed by pulling the first end 30a of the first barbed suture 30'-1 to cause the first pull region 40a to move the first surface 26a towards the interior volume 14' and by pulling the second end 30b of the first barbed suture 30'-1 to cause the second pull region 40b to move the second surface 26b towards the interior volume 14', and by pulling the first end 30a of the second barbed suture 30'-2 to cause the third pull region 40c to move the third surface 26c towards the interior volume 14' and by pulling the second end 30b of the second barbed suture 30'-2 to cause the fourth pull region 40d to move the fourth surface 26d towards the interior volume 14'.

The step of pulling the first and second bi-directional barbed sutures 30'-1 and 30'-2, respectively, that have been inserted in the muscularis 26 (or in the muscularis 26 and the serosa 28) each in series sequential uncrossed configuration may be implemented or performed by pulling the first end 30a of the first barbed suture 30'-1 to cause the first pull region 40a to move the first surface 26a towards the interior volume 14' and by pulling the second end 30b of the first barbed suture 30'-1 to cause the second pull region 40b to move the second surface 26b towards the interior volume 14' may be performed until the first and second surfaces 26a and 26b, respectively, move to an at least proximate position one to another as illustrated in FIG. 15.

Additionally, the step of pulling the first and second bi-directional barbed sutures 30'-1 and 30'-2, respectively, that have been inserted in the muscularis 26 (or in the muscularis 26 and the serosa 28) each in series sequential uncrossed configuration may be implemented or performed by pulling the first end 30a of the second barbed suture 30'-2 to cause the third pull region 40c to move the third surface 26c towards the interior volume 14' and by pulling the second end 30b of the second barbed suture 30'-2 to cause the fourth pull region 40d to move the fourth surface 26d towards the interior volume 14' until the third and fourth surfaces 26c and 26d, respectively, move to an at least proximate position one to another reducing thereby the interior volume 14' of the stomach 14.

FIGS. 16-18 illustrate still further embodiments of the method of gastroplasty according to the present disclosure. For simplicity, and given the foregoing descriptions of the embodiments of the method of gastroplasty illustrated in FIGS. 5-15, only the steps of inserting the barbed suture 30 are illustrated and described. Those skilled in the art will recognize how the steps of pulling the barbed suture 30 may be performed to achieve the final configuration of the gastroplasty.

More particularly, FIG. 16 is a cross-section view of the stomach 14 at the locations for vertical gastroplasties and a horizontal gastroplasty according to one embodiment of the method of the present disclosure wherein the method is performed by the step of inserting mono-directional barbed suture 30" (see FIG. 3B), having an anchor 60 at the trailing end 36b of the suture 30", in a series sequential configuration partially through the muscularis 26 and partially through both the muscularis 26 and the serosa 28 to create first pull region 40a, second pull region 40b, third pull region 40c, fourth pull region 40d and fifth pull region 40e, respectively, therein, before reducing the interior volume 14' of the stomach 14 wherein the anchor 60 is disposed in the interior volume 14' of the stomach 14.

In a similar manner as with respect to the method of gastroplasty described in relationship to FIG. 16, FIG. 17 is a cross-section view of the stomach 14 at the locations for vertical gastroplasties and a horizontal gastroplasty according to one embodiment of the method of the present disclosure wherein the method is performed by the step of inserting mono-directional barbed suture 30" (see FIG. 3B), having an anchor 60 at the trailing end 36b of the suture 30", and securely jointed thereto, in a series sequential configuration partially through the muscularis 26 and partially through both the muscularis 26 and the serosa 28 to create first pull region 40a, second pull region 40b, third pull region 40c and fourth pull region 40d, respectively, therein, before reducing the interior volume 14' of the stomach 14 wherein the anchor 60 is disposed externally to the stomach 14.

In this method, those skilled in the art will recognize that the insertion of the first end 36a of barbed suture 30" may begin laparoscopically from a position 28' in the serosa that is external to the stomach 14 while the remaining steps of inserting the barbed suture 30" through the muscularis 26 and serosa 28 may be performed through the esophagus 12 (see FIG. 1).

In FIGS. 16 and 17, the anchor 60 may include a pledget or a buttress or an end effector or other suitable restraint as known to or conceivable by those skilled in the art. The embodiments of the method of gastroplasty illustrated in FIGS. 16 and 17 are not limited in this context. In either of the embodiments of the method of gastroplasty illustrated in FIGS. 16 and 17, the anchor 60 serves to limit further forward movement of the barbed suture 30" in the direction of insertion thereof.

FIG. 18 is a cross-section view of the stomach at the locations for vertical gastroplasties and a horizontal gastroplasty according to one embodiment of the method of the present disclosure wherein the method is performed by inserting the first end 30'''a of substantially mono-directional barbed suture 30"' (see FIG. 3C) with bi-directional trailing end 30'''b in a series sequential configuration through both the muscularis 26 and the serosa 28 to create first pull region 40a, second pull region 40b, third pull region 40c and fourth pull region 40d, respectively, therein, before reducing the interior volume 14' of the stomach 14, including a cross-pull configuration forming overlap 42b at second pull region 40b.

In this method, the opposing direction of the barbs 32 on the surface of the trailing end 30"'b serve an analogous function to the anchor 60 described above with respect to FIGS. 16 and 17 in that the barbs 32 on the surface of the trailing end 30'''b serve to limit further forward movement of the barbed suture 30"' in the direction of insertion thereof.

Referring again particularly to FIGS. 2 and 3, the steps of the above-described methods of gastric reduction that include inserting the first end 30a of the one or more barbed sutures 30 through the first surface 26a wherein the first end 30a enters the first surface 26a and exits the first surface 26a of the muscularis 26 and penetrates into the interior volume 14' of the stomach 14; inserting the first end 30a that has penetrated the interior volume 14' of the stomach 14 into the second surface 26b of the muscularis 26, wherein the first end 30a enters the second surface 26b and exits the second surface 26b of the muscularis 26 and penetrates into the interior volume 14' of the stomach 14; and pulling the first end 30a until the first and second surfaces 26a and 26b, respectively, of the muscularis 26 move to an at least proximate position one to another, thereby reducing the interior volume 14' of the stomach 14, are performed wherein the first end 30a enters the first and second surfaces 26a and 26b, respectively, of the muscularis 26 through the mucosa layer 20a and the submucosa layer 20b and exits the first and second surfaces 26a and 26b, respectively, either consecutively or individually, through the submucosa layer 20b and the mucosa layer 20a.

Those skilled in the art will recognize that, in a similar manner, the first end 30a enters the third and fourth surfaces 26c and 26d, respectively, either consecutively or individually, or additional surfaces of the muscularis 26 through the mucosa layer 20a and the submucosa layer 20b and exits the third and fourth surfaces 26c and 26d, respectively, through the submucosa layer 20b and the mucosa layer 20a.

As previously mentioned, those skilled in the art will recognize that the one or more barbed sutures 30 utilized in the above-described methods of gastric reduction according to the present disclosure may be formed of a suitable absorbable monofilament structure that includes bi-directional barbs formed thereon. Alternatively, as described above, the sutures 30 may be non-absorbable or may be braided in a multifilament structure.

It will be understood that various modifications may be made to the embodiments shown herein. Therefore, the above description should not be construed as limiting, but merely as exemplifications of multiple embodiments. Those skilled in the art will envision other modifications within the scope and spirit of the claims appended hereto.

## Claims

1. Use of a degradable material selected from the group consisting of polyesters, polyorthoesters, polymer drugs, polydroxybutyrates, proteins, cat gut, carbonates, homopolymers thereof, copolymers thereof, and combinations thereof, and/or a polymer selected from the group consisting of polyolefins; polyethylene glycols; polyethylene oxides; copolymers of polyethylene and polypropylene; polyisobutylene and ethylene-alpha olefin copolymers; fluorinated polyolefins; polyamides; polyamines; polyimines; polyesters; polyethers; polybutester; polytetramethylene ether glycol; 1,4-butanediol; polyurethanes; acrylic polymers; methacrylics; vinyl halide polymers and copolymers; polyvinyl alcohols; polyvinyl ethers; polychlorofluoroethylene; polyacrylonitrile; polyaryletherketones; polyvinyl ketones; polyvinyl aromatics; polyvinyl esters; copolymers of vinyl monomers with each other and olefins; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof,
in the manufacture of a barbed suture for use in gastric reduction, said gastric reduction comprising the steps of:
inserting an oral-gastric device into the interior volume of the stomach of a subject;
inserting through the esophagus at least one barbed suture wherein the inserting of the oral-gastric device enables the at least one barbed suture to be positioned in proximity to the muscularis of the stomach of a subject, the at least one barbed suture having a first end, the first end having a pointed configuration, and a second end, wherein the muscularis comprises at least first and second surfaces, the first and second surfaces in at least partially interfacing relationship to one another with respect to the interior volume of the stomach;
inserting at least one of the first end and the second end of the at least one barbed suture through the muscularis to form at least first and second pull regions; and
pulling the at least one of the first end and the second end of the at least one barbed suture to cause the first pull region to move the first surface towards the interior volume and to cause the second pull region to move the second surface towards the interior volume, reducing thereby the interior volume of the stomach.

2. Use according to claim 1,
wherein the step of pulling the at least one of the first end and the second end of the at least one barbed suture to cause the first pull region to move the first surface towards the interior volume and to cause the second pull region to move the second surface towards the interior volume is performed until the first and second surfaces move to an at least proximate position one to another, reducing thereby the interior volume of the stomach.

3. Use according to claim 1 or claim 2,
wherein the step of inserting the at least one of the first end and the second end of the at least one barbed suture through the muscularis to form at least first and second pull regions is performed by inserting the first end of the at least one barbed suture through the muscularis and the serosa to form at least first and second pull regions.

4. Use according to any preceding claim,
wherein the step of inserting the at least one of the first end and the second end of the at least one barbed suture through the muscularis to form at least first and second pull regions is performed by inserting the first end of the at least one barbed suture through the first surface,
wherein the first end of the at least one barbed suture enters the first surface of the muscularis and exits the first surface of the muscularis and penetrates into the interior volume of the stomach; and
inserting the first end of the at least one barbed suture that has penetrated the interior volume of the stomach into the second surface of the muscularis,
wherein the first end of the at least one barbed suture enters the second surface of the muscularis and exits the second surface of the muscularis and penetrates into the interior volume of the stomach.

5. Use according to claim 4, wherein the step of inserting the at least one of the first end and the second end of the at least one barbed suture through the muscularis to form at least first and second pull regions is performed by inserting a bi-directional barbed suture in the muscularis layers in a series sequential uncrossed configuration before reducing the interior volume of the stomach.

6. Use according to claim 5 wherein the step of pulling the at least one of the first end and the second end of the at least one barbed suture to cause the first pull region to move the first surface towards the interior volume and to cause the second pull region to move the second surface towards the interior volume is performed by pulling the barbed suture that has been inserted in the muscularis layers in a series sequential uncrossed configuration to reduce the interior volume of the stomach.

7. Use according to claim 4, wherein the step of inserting the at least one of the first end and the second end of the at least one barbed suture through the muscularis to form at least first and second pull regions is performed by inserting a bi-directional barbed suture in the muscularis layers in a cross-pull series sequential configuration before reducing the interior volume of the stomach.

8. Use according to claim 7, wherein the step of pulling the at least one of the first end and the second end of the at least one barbed suture to cause the first pull region to move the first surface towards the interior volume and to cause the second pull region to move the second surface towards the interior volume is performed by pulling the barbed suture that has been inserted in the muscularis in a cross-pull series sequential configuration to reduce the interior volume of the stomach.

9. Usse according to claim 4, wherein the step of inserting the at least one of the first end and the second end of the at least one barbed suture through the muscularis to form at least first and second pull regions is performed by inserting a bi-directional barbed suture in the muscularis in a partially uncrossed and partially cross-pull series sequential configuration before reducing the interior volume of the stomach;
preferably wherein the step of pulling the at least one of the first end and the second end of the at least one barbed suture to cause the first pull region to move the first surface towards the interior volume and to cause the second pull region to move the second surface towards the interior volume is performed by pulling the at least one barbed suture that has been inserted in the muscularis in a partially uncrossed and partially cross-pull series sequential configuration to reduce the interior volume of the stomach.

10. Use according to claim 4, wherein the step of inserting the at least one of the first end and the second end of the at least one barbed suture through the muscularis to form at least first and second pull regions is performed by inserting first and second bi-directional barbed sutures in the muscularis each in a series sequential configuration and in traversing paths before reducing the interior volume of the stomach;
preferably wherein the step of pulling the at least one of the first end and the second end of the at least one barbed suture to cause the first pull region to move the first surface towards the interior volume and to cause the second pull region to move the second surface towards the interior volume is performed by pulling the first and second bi-directional barbed sutures that have been inserted in the muscularis each in series sequential configuration and in traversing paths to reduce the interior volume of the stomach.

11. Use according to claim 4, wherein the step of inserting the at least one of the first end and the second end of the at least one barbed suture through the muscularis to form at least first and second pull regions is performed by inserting a mono-directional barbed suture having an anchor at the trailing end of the suture in a series sequential configuration partially through the muscularis and partially through both the muscularis and the serosa before reducing the interior volume of the stomach wherein the anchor is disposed in the interior volume of the stomach;
preferably wherein the step of pulling the at least one of the first end and the second end of the at least one barbed suture to cause the first pull region to move the first surface towards the interior volume and to cause the second pull region to move the second surface towards the interior volume is performed by pulling the mono-directional barbed suture having an anchor at the trailing end of the suture that has been inserted in a series sequential configuration partially through the muscularis and partially through both the muscularis and the serosa reducing thereby the interior volume of the stomach wherein the anchor is disposed in the interior volume of the stomach.

12. Use according to claim 4, wherein the step of inserting the at least one of the first end and the second end of the at least one barbed suture through the muscularis to form at least first and second pull regions is performed by inserting a mono-directional barbed suture having an anchor at the trailing end of the suture in a series sequential configuration partially through the muscularis and partially through both the muscularis and the serosa before reducing the interior volume of the stomach wherein the anchor is disposed externally to the stomach;
preferably.wherein the step of pulling the at least one of the first end and the second end of the at least one barbed suture to cause the first pull region to move the first surface towards the interior volume and to cause the second pull region to move the second surface towards the interior volume is performed by pulling the mono-directional barbed suture having an anchor at the trailing end of the suture that has been inserted in a series sequential configuration partially through the muscularis and partially through both the muscularis and the serosa reducing thereby the interior volume of the stomach wherein the anchor is disposed externally to the stomach.

13. Use according to claim 4, wherein the step of inserting the at least one of the first end and the second end of the at least one barbed suture through the muscularis to form at least first and second pull regions is performed by inserting a substantially mono-directional barbed suture with a bi-directional trailing end in a series sequential configuration through both the muscularis and the serosa before reducing the interior volume of the stomach;
preferably wherein the step of pulling the at least one of the first end and the second end of the at least one barbed suture to cause the first pull region to move the first surface towards the interior volume and to cause the second pull region to move the second surface towards the interior volume is performed by pulling the substantially mono-directional barbed suture with a bi-directional trailing end in a series sequential configuration through both the muscularis and the serosa reducing thereby the interior volume of the stomach.

14. Use according to any preceding claim, wherein the pointed configuration of the first end of the at least one barbed suture is availed by a surgical needle being removably attached to the at least one barbed suture at the first end thereof.

15. Use according to any preceding claim, wherein the pointed configuration of the first end of the at least one barbed suture is availed by a surgical needle being removably attached to the at least one barbed suture via an aperture formed in the surgical needle at a central portion thereof.
